# EUROPEAN PATENT APPLICATION

(11) **EP 1 541 176 A1**
(43) Date of publication of application: **15.06.2005**
(21) Application number: 03791199.7
(22) Date of filing: 07.08.2003
(51) Int. Cl.: A61K 47/32, A61K 31/48, A61K 9/70, A61K 47/08

(54) **ADHESIVE PATCH**

(30) Priority: 28.08.2002 JP 2002249417
(71) Applicant: HISAMITSU PHARMACEUTICAL CO. INC., Tosu-shi, Saga-ken 841-0017 (JP)
(72) Inventor: TERAHARA, Takaaki, c/o Tsukuba Lab. of Hisamitsu, Tsukuba-shi, Ibaraki 305-0856 (JP); AIDA, Kazunosuke, c/o Tsukuba Lab. of Hisamitsu, Tsukuba-shi, Ibaraki 305-0856 (JP); TOSHIMITSU, Arata, c/o Tsukuba Lab. of Hisamitsu, Tsukuba-shi, Ibaraki 305-0856 (JP); HIGO, Naruhito, c/o Tsukuba Lab. of Hisamitsu, Tsukuba-shi, Ibaraki 305-0856 (JP)
(74) Representative: Grünecker, Kinkeldey, Stockmair & Schwanhäusser Anwaltssozietät
(86) International application number: PCT/JP2003/010091
(87) International publication number: WO 2004/019987

(57) **Abstract**

A patch comprising a backing layer and an adhesive layer disposed on the backing layer and compounded with an adhesive base agent and pergolide and/or a pharmaceutically acceptable salt thereof, wherein the adhesive base agent comprises an acrylic polymer including no substantial carboxyl group and hydroxyl group and having self-adhesion properties and a rubber polymer, and the weight ratio of the content of the acrylic polymer to the content of the rubber polymer is from 1:1 to 1:9.

## Description

### Technical Field

The present invention relates to a patch, and more specifically to a patch containing pergolide.

### Background Art

Conventionally, an oral administration method using a tablet, a capsule, a syrup or the like has been known as a method for administering pharmaceutical. However, in recent years an approach has been tried in which drugs are transdermally administrated using a patch. The administration method using a patch can overcome problems associated with the oral administration method and, in addition, has advantages such as a decrease in the administration frequency, improvement of compliance, ease of administration as well as ease of discontinuation thereof. Therefore, use of a patch is considered promising as a useful administration method for a drug, especially in a case where patients are elderly or children.

However, the stratum corneum of the normal skin has a barrier function for inhibiting exogenous materials from penetrating into the body. Due to the barrier function, compounded medicinal ingredients are often not transdermally absorbed sufficiently when conventional patches are used. Further, since the stratum corneum has a high lipid solubility, the skin permeability of a drug is generally extremely low.

Therefore, in order to enhance the transdermal absorption properties of a drug in the transdermal administration method, studies are being carried out concerning the composition of an adhesive agent for use in a patch and the like. As one part of such studies, a patch has been proposed that uses polymer material such as acrylic polymer or rubber polymer as an adhesive base agent (JP-A-4-266821, JP-A-9-301854 and the like).

### Disclosure of the Invention

However, even when the aforementioned conventional patches are used, it can not necessarily be said that the skin permeability of a drug is sufficient. In addition, in these conventional patches, due to a fact that patch properties such as a cohesion property and an adhesion property of the adhesive layer are degraded when a transdermal absorption property of a drug is enhanced, it is very difficult to satisfy all the characteristic features that are required for a patch. Further, the properties of drugs for use in a patch vary depending on the kinds thereof and, on the other hand, the compatibility between adhesive base agents and specific drugs has not yet been investigated sufficiently.

The present invention was achieved in consideration of the problems included in the aforementioned conventional technique, and the object of the invention is to provide a patch that enables a high level to be achieved for both the skin absorption properties of the drug and the patch properties when pergolide and/or a pharmaceutically acceptable salt thereof is contained as a drug.

The present inventors carried out concentrated studies to achieve the aforementioned object, and found that many of the acrylic polymers among polymer materials used in the conventional patch have a carboxyl group (-COOH) or a hydroxyl group (-OH) in the molecule as a functional group for crosslinking, and that it is very difficult to achieve compatibility between the skin permeability of a drug and the patch properties when using this kind of acrylic polymer. As the result of further studies based on this finding, we found that in a patch containing pergolide and/or a pharmaceutically acceptable salt thereof as a drug, the aforementioned problems could be overcome by incorporating an acrylic polymer without a substantial carboxyl group and hydroxyl group in the molecular and with self-adhesion properties, and a rubber polymer in an adhesive layer at a specified ratio, respectively, to accomplish the invention.

In other words, the patch of this invention is characterized in that it comprises a backing layer and an adhesive layer disposed on the backing layer and compounded with an adhesive base agent and pergolide and/or a pharmaceutically acceptable salt thereof, wherein the adhesive base agent comprises an acrylic polymer including no substantial carboxyl group and hydroxyl group in the molecule and having self-adhesion properties, and a rubber polymer, and a weight ratio of the content of the acrylic polymer is to the content of the rubber polymer from 1:1 to 1:9.

In this connection, the phrase "with self-adhesion properties" means that, when a polymer is formed into a film shape and a tack test (the rolling ball method, JIS Z 0237) is carried out using the film at normal temperature, the ball stops on the film.

In the patch of the invention, it is preferable that the adhesive base agent further comprises a basic nitrogen-including polymer including a basic nitrogen and having no self-adhesion property, and that the weight ratio of the total content of the acrylic polymer and the rubber polymer to the content of the basic nitrogen-including polymer is from 1:1 to 9:1.

In the patch of the invention, preferably the basic nitrogen-including polymer is at least one kind selected from methyl methacrylate - butyl methacrylate - dimethylaminoethyl methacrylate terpolymer and polyvinyl acetal diethylamino acetate.

In the patch of the invention, preferably the adhesive layer further comprises an alicyclic saturated hydrocarbon resin tackifier, wherein the weight ratio of the total content of the acrylic polymer and the rubber polymer to the tackifier is from 1:1 to 1:9.

Further, in the patch of the invention, it is preferable that the acrylic polymer is at least one kind selected from: copolymer of polyacrylate including at least one selected from 2-ethylhexyl acrylate, butyl acrylate, diacetone acrylamide and tetraethylene glycol dimethacrylate, and polymethyl methacrylate; 2-ethylhexyl acrylate - N-vinyl-2-pyrrolidone - 1,6-hexane glycol dimethacrylate terpolymer; and 2-ethylhexyl acrylate - vinyl acetate copolymer.

In the patch of the invention, preferably the rubber polymer is at least one kind selected from styrene-isoprene-styrene block copolymer, styrene-butadiene-styrene block copolymer, styrene-butadiene rubber, polyisobutylene, isoprene rubber and silicone rubber, and more preferably it is styrene-isoprene-styrene block copolymer.

In the patch of the invention, it is preferable that the acrylic polymer is at least one kind selected from 2-ethylhexyl acrylate - N-vinyl-2-pyrrolidone - 1,6-hexane glycol dimethacrylate terpolymer and 2-ethylhexyl acrylate - vinyl acetate copolymer; and that the rubber polymer is styrene-isoprene-styrene block copolymer.

Further, in the patch of the invention, preferably a mesylate salt of pergolide is compounded in the adhesive layer.

Furthermore, in the patch of the invention, preferably the adhesive layer further comprises an organic acid, and more preferably the organic acid is acetic acid and/or a pharmaceutically acceptable salt thereof.

### Best Mode for Carrying Out the Invention

Hereinafter, preferable embodiments of the invention will be described in detail.

The patch of the invention is a patch comprising a backing layer and an adhesive layer disposed on the backing layer and compounded with an adhesive base agent and pergolide and/or a pharmaceutically acceptable salt thereof, wherein the adhesive base agent comprises an acrylic polymer including no substantial carboxyl group and hydroxyl group and having self-adhesion properties, and a rubber polymer at a weight ratio of the content of the acrylic polymer to the content of the rubber polymer that is from 1:1 to 1:9.

As the backing layer for use in the patch of the invention, any one may be used without particular limitation insofar as it can support the adhesive layer, and a stretchable or an unstretchable backing layer may be used. Among them, one selected from woven cloth, nonwoven cloth and knitted cloth that have moisture permeability is preferable. Use of a backing layer having moisture permeability allows sweat accumulated between an affected part and the patch upon sticking to effuse effectively and makes it possible to prevent stuffiness and skin stimulation provided by the sweat. As such backing layer, specific examples include cloth and nonwoven cloth, polyurethane, polyester, polypropylene, polyvinyl acetate, polyvinylidene chloride, polyethylene, polyethylene terephthalate and aluminum sheet, one made up to woven cloth, nonwoven cloth or knitted cloth from synthetic or natural fiber such as nylon, acrylic, cotton, rayon or acetate or a complex thereof, and further conjugated material of these and film having moisture permeability and the like. Among them, knitted cloth made of polyester is preferably used from the point of safeness, versatility and stretchability.

Thickness of the backing layer according to the invention is not particularly limited, but a thickness in a range of from 5 to 1000 µm is preferable. A thickness of the backing layer lower than the lowest limit described above tends to decrease operation easiness upon sticking the patch and, on the other hand, that higher than the highest limit described above tends to decrease production easiness in the production process of the patch due to difficulty of cutting the backing layer or the patch, or the like.

In a patch of the invention, an adhesive layer compounded with an adhesive base agent and pergolide and/or a pharmaceutically acceptable salt thereof is disposed on the backing layer. The adhesive base agent according to the invention is an adhesive base agent that comprises an acrylic polymer including no substantial carboxyl group and hydroxyl group in the molecule and having self-adhesion properties (hereinafter, simply referred to as "acrylic polymer" depending on the case), and a rubber polymer.

In this connection, the acrylic polymer including no substantial carboxyl group (carboxylic acid group, - COOH) and hydroxyl group (-OH) in the molecule according to the invention means an acrylic polymer that has no carboxyl group or hydroxyl group in the molecule thereof that may become a functional group upon crosslinking. These polymers may be obtained by polymerizing monomers that have no carboxyl group and hydroxyl group. Examples of such monomers include methyl acrylate, ethyl acrylate, propyl acrylate, amyl acrylate, butyl acrylate, 2-ethylbutyl acrylate, hexyl acrylate, heptyl acrylate, octyl acrylate, nonyl acrylate, 2-ethylhexyl acrylate, decyl acrylate, dodecyl acrylate, tridecyl acrylate and, in addition, (meth)acrylic esters corresponding to targeted acrylic polymers, and the like.

Preferable examples of the acrylic polymer according to the invention include:
(A1) copolymer of polyacrylate including at least one selected from 2-ethylhexyl acrylate, butyl acrylate, diacetone acrylamide and tetraethylene glycol dimethacrylate, and polymethyl methacrylate,
(A2) 2-ethylhexyl acrylate - N-vinyl-2-pyrrolidone - 1,6-hexaneglycol dimethacrylate terpolymer, and
(A3) 2-ethylhexyl acrylate - vinyl acetate copolymer.

Examples of commercial acrylic polymers including no substantial carboxyl group and hydroxyl group and having self-adhesion properties include DURO-TAK87-2097 (having no functional group), DURO-TAK87-2194 (having no functional group) and DURO-TAK87-4098 (having no functional group) supplied by National Starch & Chemical, and the like. Among them, use of 2-ethylhexyl acrylate - N-vinyl-2-pyrrolidone - 1,6-hexaneglycol dimethacrylate terpolymer and/or 2-ethylhexyl acrylate - vinyl acetate copolymer is preferable because both the skin permeability of the drug and the patch properties tend to be enhanced more thereby. One kind of these acrylic polymers may be used independently or two or more kinds thereof may be used in combination.

In this connection, if a monomer having a carboxyl group or a hydroxyl group should exist in the raw monomer by a small amount as impurities, or when a side reaction such as thermal degradation should occur upon polymerization in a production process of the acrylic polymer described above, a carboxyl group or a hydroxyl group derived from the impurities will be introduced in the acrylic polymer to be obtained. However, such acrylic polymer is intended to be encompassed in the acrylic polymer substantially free of both carboxyl group and hydroxyl group, insofar as it does not damages a sufficiently high skin permeability of a drug and a sufficiently high patch property, which belong to the patch of the invention.

However, it is preferable to decrease a carboxyl group and a hydroxyl group in the acrylic polymer according to the invention as far as possible, even if they are ones derived from contamination of impurities or from side reaction such as thermal degradation in the production process.

The viscosity-average molecular weight of the acrylic polymer according to the invention is preferably from 200000 to 1000000. A viscosity-average molecular weight of the acrylic polymer that is lower than the lowest limit described above tends to decrease patch properties (especially an adhesion property) and, on the other hand, that higher than the highest limit described above tends to decrease compatibility with other ingredients contained in the adhesive layer.

The term "rubber polymer" according to the invention refers to a natural or synthetic elastic polymer.

Preferable examples of such rubber polymer include:
(S1) styrene-isoprene-styrene block copolymer,
(S2) styrene-butadiene-styrene block copolymer,
(S3) styrene-butadiene rubber,
(S4) polyisobutylene,
(S5) isoprene rubber, and
(S6) silicone rubber.
Among them, use of styrene-isoprene-styrene block copolymer or polyisobutylene is more preferable because it tends to enhance both the skin permeability of the drug and the patch properties. One kind of these rubber polymers may be used independently or two or more kinds thereof may be used in combination.

Furthermore, use of at least one kind selected from 2-ethylhexyl acrylate - N-vinyl-2-pyrrolidone - 1,6-hexaneglycol dimethacrylate terpolymer and 2-ethylhexyl acrylate - vinyl acetate copolymer as the acrylic polymer, and use of styrene-isoprene-styrene block copolymer as the rubber polymer, respectively, are preferable because both the skin permeability of the drug and the patch properties are enhanced more thereby.

The viscosity-average molecular weight of the rubber polymer according to the invention is preferably 30000-2500000, and more preferably 100000-1700000. A viscosity-average molecular weight that is lower than the lowest limit described above tends to decrease patch properties (especially an adhesion property) and, on the other hand, that higher than the highest limit described above tends to decrease compatibility with other ingredients contained in the adhesive layer.

In the invention, it is necessary that the weight ratio of the content of the acrylic polymer including no substantial carboxyl group and hydroxyl group in the molecule to the content of the rubber polymer is in a range from 1:1 to 1:9. By determining the weight ratio of both contents to be within the aforementioned range, when pergolide and/or a pharmaceutically acceptable salt thereof is compounded in the adhesive layer as a drug, skin permeability of the drug is significantly enhanced and high-level physical properties can be achieved for the preparation. Furthermore, by determining the weight ratio of both contents to be within the aforementioned range, a moderate adhesibility is given to the adhesive layer, and sticking properties and skin irritating properties are improved. In this connection, when the content of the rubber polymer is less than the content of the acrylic polymer, the skin permeability of the drug will be insufficient. On the other hand, when the content of the rubber polymer is more than 9 times that of the acrylic polymer, the patch properties will be insufficient.

Although the content of the acrylic polymer according to the invention is not particularly limited insofar as the weight ratio of the content thereof to the content of the rubber polymer is in the aforementioned range, it is preferably 0.2-60 % by weight, more preferably 0.5-50 % by weight, and furthermore preferably 1-40 % by weight relative to the total amount of the adhesive base agent. A compounding amount of the acrylic polymer that is lower than the lowest limit described above tends to decrease the skin permeability of the drug and, on the other hand, that higher than the highest limit described above tends to decrease the cohesive force of the adhesive layer.

Although the compounding amount of the rubber polymer according to the invention is not particularly limited insofar as the weight ratio of the content thereof to the content of the acrylic polymer is in the aforementioned range, it is preferably 0.2-60 % by weight, more preferably 0.5-50 % by weight, and further preferably 1-40 % by weight relative to the total amount of the adhesive base agent. A compounding amount of the rubber polymer that is lower than the lowest limit described above tends to decrease the skin permeability of the drug and, on the other hand, that higher than the highest limit described above tends to decrease the adhesibility of the adhesive layer.

It is preferable that the adhesive base agent according to the invention further comprises a basic nitrogen-including polymer including basic nitrogen and having no self-adhesion property (hereinafter, simply referred to as "a basic nitrogen-including polymer"), in addition to the acrylic polymer and the rubber polymer described above. By incorporating a basic nitrogen-including polymer in the adhesive base agent, it is possible to further enhance the solubility of the drug and the patch properties. Accordingly, when pergolide and/or a pharmaceutically acceptable salt thereof is compounded in the adhesive layer, a phenomenon in which these medical agents crystallize to precipitate is prevented with greater certainty, thereby enabling the provision of a patch which endures long-term storage, has a good skin absorption property, and exerts a pharmacologic effect continually over an extended time period.

As this kind of basic nitrogen-including polymer, a polymer including a functional group such as an amino group, an amide group, an imino group or an imido group may be used. When the basic nitrogen-including polymer has an amino group, the amino group may be any of a primary, a secondary and a tertiary group. Further, when the amino group is either secondary or tertiary, substituting alkyl groups may be linear or form a cycle.

Examples of this kind of basic nitrogen-including polymer include a homopolymer or a copolymer of two or more kinds of polymerizable amines such as dialkylaminoalkyl (meth)acrylate including dimethylaminoethyl (meth)acrylate and diethylaminoethyl (meth)acrylate, and vinyl pyrrolidone, a copolymer of one kind or two or more kinds of the aforementioned polymerizable amines and another polymerizable monomer, and polyvinyl dialkylamino acetate such as polyvinyl acetal diethylamino acetate.

Examples of the monomer capable of polymerizing with polymerizable amine include methyl acrylate, ethyl acrylate, propyl acrylate, butyl acrylate, isobutyl acrylate, hexyl acrylate, octyl acrylate, 2-ethylhexyl acrylate, decyl acrylate, isodecyl acrylate, lauryl acrylate, stearyl acrylate, methyl methacrylate, ethyl methacrylate, butyl methacrylate, isobutyl methacrylate, 2-ethylhexyl methacrylate, decyl methacrylate, isodecyl methacrylate, lauryl methacrylate and stearyl methacrylate.

Among the basic nitrogen-including polymers described above, use of at least one kind selected from methyl methacrylate - butyl methacrylate - dimethylaminoethyl methacrylate terpolymer and polyvinyl acetal diethylamino acetate is preferable because it enables a higher level of compatibility with respect to the skin permeability of the drug and the patch properties. As methyl methacrylate - butyl methacrylate - dimethylaminoethyl methacrylate terpolymer and polyvinyl acetal diethylamino acetate, commercially available Eudragit E (trade name, manufactured by Röhm) and AEA (trade name, manufactured by SANKYO), respectively, and the like can be used.

The viscosity-average molecular weight of the basic nitrogen-including polymer is preferably 100000-5000000, and more preferably 1000000-3000000. A viscosity-average molecular weight of the basic nitrogen-including polymer that is lower than the lowest limit described above tends to cause the effect of adding the basic nitrogen-including polymer (especially a cohesion property) to be insufficient and, on the other hand, that higher than the highest limit described above tends to decrease compatibility with other ingredients contained in the adhesive layer.

In the adhesive base agent, the content of the basic nitrogen-including polymer is not particularly limited, but it is preferable that the weight ratio of the total content of the acrylic polymer and the rubber polymer to the basic nitrogen-including polymer is from 9:1 to 1:1. When the content of the basic nitrogen-including polymer is less than one ninth of the total content of the acrylic polymer and the rubber polymer, an effect produced by the basic nitrogen-including polymer is liable to be insufficient with respect to enhancing the skin permeability of the drug. On the other hand, when the content of the basic nitrogen-including polymer exceeds the total content of the acrylic polymer and the rubber polymer, the adhesion properties of the adhesive layer are liable to be reduced.

In the adhesive base agent, the content of the basic nitrogen-including polymer is preferably 1-30 % by weight, and more preferably 5-20 % by weight relative to the total amount of the adhesive base agent. A content of the basic nitrogen-including polymer that is lower than the lower limit described above tends to decrease the skin permeability of the drug and, on the other hand, that higher than the highest limit described above tends to decrease the adhesion properties of the adhesive layer.

In this connection, in the invention, the adhesive layer may further contain a rubber polymer such as ethylene-vinyl acetate copolymer (EVA, content of vinyl acetate: 5-60 % by weight) insofar as skin permeability of the drug and patch properties are not impaired. The content of this kind of rubber polymer is preferably 0.05-1 % by weight relative to the total amount of the compounds contained in the adhesive layer.

In the patch of the invention, pergolide and/or a pharmaceutically acceptable salt thereof is compounded in the adhesive layer as an active ingredient. When a pharmaceutically acceptable salt of pergolide is compounded, the salt may be either an inorganic salt or an organic salt, and mesylate salt (that is, pergolide mesylate) is especially preferable.

In the invention, a compounding amount of pergolide and/or a pharmaceutically acceptable salt thereof is preferably 0.1-50 % by weight, and more preferably 1-20 % by weight relative to the total amount of the compounds contained in the adhesive layer. A compounding amount of pergolide and/or a pharmaceutically acceptable salt thereof that is lower than the lowest limit described above tends to decrease the skin permeability of the drug. In contrast, a compounding amount that is higher than the highest limit described above tends to decrease physical properties, because pergolide and/or a pharmaceutically acceptable salt thereof may not completely dissolve in the adhesive layer and instead crystallize to precipitate.

The adhesive layer according to the invention is an adhesive layer comprising the aforementioned adhesive base agent and the drug and, in addition to these ingredients, it may further comprise a tackifier. Specific examples of the tackifer for use in the invention include rosin derivatives (rosin, rosin glycerine ester, hydrogenated rosin, hydrogenated rosin ester, rosin pentaerythritol ester and the like), alicyclic saturated hydrocarbon resin (Arkon P100 (manufactured by Arakawa Chemical Industries) and the like), aliphatic hydrocarbon resin (Quintone B-170 (manufactured by ZEON CORPORATION) and the like), terpene resin (Clearon P-125 (manufactured by Yasuhara Chemical) and the like), maleic acid resin and the like. Among them, hydrogenated rosin glycerine ester, aliphatic hydrocarbon resin and terpene resin are preferable, and alicyclic saturated hydrocarbon resin is especially preferable.

The compounding amount of the tackifier according to the invention is not particularly limited, but it is preferably 5-70 % by weight, more preferably 5-60 % by weight, and further preferably 10-50 % by weight relative to the total amount of the compounds contained in the adhesive layer. When a compounding amount of the tackifier is lower than the lowest limit described above, the effect produced by compounding the tackifier tends to be insufficient with respect to enhancing the adhesibility of the patch and, on the other hand, a compounding amount higher than the highest limit described above tends to increase skin irritating properties when peeling off the patch.

In the case where an alicyclic saturated hydrocarbon resin is used as a tackifier, it is preferable that the weight ratio of the total content of the acrylic polymer and the rubber polymer to the content of the tackifier is from 1:1 to 1:9. When the respective contents of the acrylic polymer, the rubber polymer and the tackifier satisfy the condition described above, both the skin permeability of pergolide and/or a pharmaceutically acceptable salt thereof and the patch properties are enhanced to a greater degree and adhesibility is also enhanced more, whereby a patch can be obtained in which sticking properties and skin irritating properties have been further improved.

In the invention, the adhesive layer further comprises, preferably, an organic acid. Examples of the organic acid include aliphatic (mono, di, tri) carboxylic acids (acetic acid, propionic acid, citric acid (including anhydrous citric acid), isobutyric acid, caproic acid, caprylic acid, lactic acid, maleic acid, pyruvic acid, oxalic acid, succinic acid, tartaric acid and the like), aromatic carboxylic acids (phthalic acid, salicylic acid, benzoic acid, acetyl salicylic acid and the like), alkyl sulfonic acids (methane sulfonic acid, ethane sulfonic acid, propyl sulfonic acid, butane sulfonic acid, polyoxyethylenealkylether sulfonic acid and the like), alkyl sulfonic acid derivatives (N-2-hydroxyethylpiperidine-N'-2-ethane sulfonic acid and the like), cholic acid derivatives (dehydrocholic acid and the like), and salts thereof (for example, alkali metal salts such as sodium salts), and the like. Among these organic acids, carboxylic acids and salts thereof are preferable, and acetic acid, sodium acetate and citric acid are especially preferable. One kind of these organic acids may be used independently or a mixture of two or more kinds thereof may be used.

In the adhesive layer according to the invention, the content of the organic acid is not particularly limited, but it is preferably 0.01-20 % by weight, more preferably 0.1-15 % by weight, and further preferably 0.1-10 % by weight relative to the total amount of the compounds contained in the adhesive layer. When a content of the organic acid is lower than the lowest limit described above, the effect produced by the organic acid tends to be insufficient with respect to enhancing the skin permeability of the drug and, on the other hand, a content that is higher than the highest limit described above tends to increase skin irritating properties.

The adhesive layer according to the invention may further comprise an absorption enhancer. As the absorption enhancer, compounds conventionally recognized to have an absorption enhancing effect at the skin may be used, more specifically, the compounds include fatty acids, aliphatic alcohols, fatty acid esters, fatty acid amides, and fatty acid ethers having 6 to 20 carbons, aromatic organic acids, aromatic alcohols, aromatic organic acid esters and ethers, which may be saturated or unsaturated, and also may be linear, branched or cyclic. Further, in the invention, lactic acid esters, acetic acid esters, monoterpenes, sesquiterpenes, Azone, Azone derivatives, glycerin fatty acid esters, propylene glycol fatty acid esters, sorbitan fatty acid esters (Span), polysorbates (Tween), polyethylene glycol fatty acid esters, polyoxyethylene hydrogenated castor oils (HCO), polyoxyethylene alkyl ethers, sucrose fatty acid esters or vegetable oils may be used as an absorption enhancer. Among these absorption enhancers, preferable examples include caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, lauric acid diethanol amide, isopropyl myristate, myristyl myristate, octyldodecyl myristate, cehyl palmitate, salicylic acid, methyl salicylate, salicylic acid ethylene glycol, cinnamic acid, methyl cinnamate, cresol, cethyl lactate, lauryl lactate, ethyl acetate, propyl acetate, geraniol, thymol, eugenol, terpineol, L-menthol, borneol, d-limonene, isoeugenol, isoborneol, nerol, dl-camphor, glycerin monocaprirate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pirotiodecane and olive oil. Of these, lauryl alcohol, myristyl alcohol, isostearyl alcohol, lauric acid diethanol amide, glycerin monocaprirate, glycerin monocaprate, glycerin monooleate, sorbitan monolaurate, propylene glycol monolaurate, polyoxyethylene lauryl ether and pirotiodecane are more preferable. One kind of these absorption enhancers may be used independently, or two or more kinds thereof may be used in combination.

In the adhesive layer according to the invention, while the content of the absorption enhancer is not particularly limited, it is preferably 0.01-20 % by weight, more preferably 0.05-10 % by weight, and further preferably 0.1-5 % by weight relative to the total amount of the compounds contained in the adhesive layer. When a content of the absorption enhancer is lower than the lowest limit described above, the effect produced by the absorption enhancer tends to be insufficient with respect to enhancing the skin permeability of the drug and, on the other hand, a content that is higher than the highest limit described above tends to increase skin irritating properties such as edema.

The adhesive layer according to the invention may further comprise a plasticizer. Specific examples of the plasticizer include petroleum oils (paraffin process oils, naphthene process oils, aromatic process oils and the like), squalane, squalene, vegetable oils (olive oil, camellia oil, castor oil, tall oil, peanut oil), silicone oil, liquid oils (polybutane, liquid isoprene rubber), liquid fatty acid esters (isopropyl myristate, hexyl laurate, diethyl sebacate, diisopropyl sebacate and the like), diethylene glycol, polyethylene glycol, propylene glycol, dipropylene glycol, triacetin, triethyl citrate, crotamiton and the like. Among these plasticizers, liquid paraffin, liquid polybutene, crotamiton, diethyl sebacate and hexyl laurate are especially preferable. One kind of these plasticizers may be used independently or two or more kinds thereof may be used in combination.

In the adhesive layer according to the invention, while the content of the plasticizer is not particularly limited, it is preferably 5-70 % by weight, more preferably 10-60 % by weight, and further preferably 10-50 % by weight relative to the total amount of the compounds contained in the adhesive layer. When the content of the plasticizer is lower than the lower limit described above, the effect produced by compounding the plasticizer tends to be insufficient with respect to enhancing the cohesive force of the patch and, on the other hand, when the content is higher than the highest limit described above the skin permeability of the drug tends to be insufficient.

Further, in the invention, the adhesive layer may be incorporated with an antioxidant, a filler, an ultraviolet absorbent or the like according to need.

Preferable examples of the antioxidant according to the invention include tocopherols and ester derivatives of these, ascorbic acid, ascorbyl stearate, nordihydroguaiaretic acid, dibutylhydroxytoluene (BHT) and butylhydroxyanisol.

Preferable examples of the filler include calcium carbonate, magnesium carbonate, silicates (such as aluminum silicate and magnesium silicate), silicic acid, barium sulfate, calcium sulfate, calcium zincate, zinc oxide and titanium oxide.

Preferable examples of the ultraviolet absorbent include p-aminobenzoic acid derivatives, anthranilic acid derivatives, salicylic acid derivatives, coumarin derivatives, amino acid-series compounds, imidazoline derivatives, pyrimidine derivatives and dioxane derivatives.

In the adhesive layer according to the invention, the respective contents of the antioxidant, the filler and the ultraviolet absorbent are not particularly limited, but the total amount of the contents of the antioxidant, the filler and the ultraviolet absorbent is preferably 0-10 % by weight, more preferably 0-5 % by weight, and further preferably 0-2 % by weight relative to the total amount of the compounds contained in the adhesive layer.

A manufacturing method for forming the adhesive layer having the aforementioned constitution on the backing layer is not particularly limited and, for example, the patch of the invention can be obtained by thermally melting a mixture of the adhesive base agent, pergolide and/or a pharmaceutically acceptable salt thereof and other ingredients described above that are added according to need, and then coating the molten mixture on the backing layer. When the patch of the invention further comprises release liner on the adhesive layer, the thermally molten mixture is coated on the release liner followed by laying the backing layer on the coated side, or the thermally molten mixture is coated on the backing layer followed by laying the release liner on the coated side, to obtain the patch of the invention. Furthermore, instead of thermally melting the mixture described above, it is also possible to use a coating liquid prepared by dissolving the mixture in a solvent such as toluene, hexane or ethyl acetate to obtain the patch of the invention.

The patch of the invention may be a patch provided with one adhesive layer, or one provided with two or more adhesive layers insofar as they do not impair the skin permeability of pergolide and/or a pharmaceutically acceptable salt thereof.

Although the thickness of the adhesive layer according to the invention is not particularly limited, it is preferably 20-200 µm. When the thickness of the adhesive layer is lower than the lowest limit described above the skin permeability of the drug tends to be insufficient and, on the other hand, a thickness that exceeds the highest limit described above tends to generate a phenomenon (adhesive agent remaining) in which the adhesive agent remains adhered to the skin after application.

Furthermore, when the patch of the invention comprises release liner, specific examples of the release liner include film made of polyester such as polyethylene terephthalate, polyvinyl chloride, polyvinylidene chloride or the like, and laminate film of bond paper and polyolefin. In these release liners, it is preferable to provide silicone treatment to the side contacting the adhesive layer, because this facilitates ease of operation when peeling the release liner off the adhesive agent.

Hereunder, the invention is explained more specifically on the basis of Examples and Comparative Examples, however the invention is not limited to these examples.

### [Example 1]

Pergolide mesylate, acetic acid, sodium acetate, sorbitan monolaurate, isostearyl alcohol and liquid paraffin were charged in a mortar and mixed sufficiently. The mixture was added to a mixed liquid consisting of 2-ethylhexyl acrylate - vinyl acetate copolymer (polymer (A)), styrene-isoprene-styrene block copolymer (polymer (B)), methyl methacrylate - butyl methacrylate - dimethylaminoethyl methacrylate terpolymer (Eudragit E, polymer (C)), an alicyclic saturated hydrocarbon resin, heptane and ethyl acetate, to prepare a coating liquid for an adhesive layer. The contents of the respective ingredients (values relative to the total amount of the compounds excluding heptane and ethyl acetate), the total content of the polymers (A) and (B), and the weight ratio of the content of the polymer (A) to the content of the polymer (B) in the obtained coating liquid are listed in Table 1. In this connection, in this Example and Examples 2-6 and Comparative Examples 1-10 described later, content of the surfactant (5.0 % by weight) means the total content of sorbitan monolaurate (2.0 % by weight) and isostearyl alcohol (3.0 % by weight).

Next, the obtained coating liquid was applied on a release liner made of polyethylene terephthalate and dried to remove the solvent to form an adhesive layer. Then, a backing layer in the form of knitted cloth made of polyester was laminated to the adhesive layer to obtain the desired patch.

### [Examples 2-3, Comparative Examples 1-2]

In Examples 2-3 and Comparative Examples 1-2, patches were prepared in the same way as Example 1 except that the respective contents of 2-ethylhexyl acrylate - vinyl acetate copolymer and styreneisoprene-styrene block copolymer were determined as listed in Tables 1 and 2.

### [Examples 4-6, Comparative Examples 3-4]

In Examples 4-6 and Comparative Examples 3-4, patches were prepared in the same way as Example 1 except that for each of the Examples and Comparative Examples methyl methacrylate - butyl methacrylate - dimethylaminoethyl methacrylate terpolymer was not used and the composition of the coating liquids for the adhesive layer was determined as listed in Tables 1 and 2.

### [Comparative Examples 5-10]

In Comparative Examples 5-10, patches were prepared in the same way as Example 1 except that for each of the Comparative Examples an acrylic polymer having a hydroxyl group (DURO-ATK87-2287) or an acrylic polymer having a carboxyl group (DURO-ATK87-2852) was used in place of 2-ehylhexyl acrylate - vinyl acetate copolymer and the composition of the coating liquids for the adhesive layer was determined as listed in Tables 2 and 3.

### (Skin permeability test)

The following tests were carried out using the respective patches obtained in Examples 1-6 and Comparative Examples 1-10.

First, dorsal skin of a hairless mouse was extirpated and the skin was set to a flow-through cell, and the periphery of a receptor layer thereof was circulated with water at 37 °C, so that the dermis side became a receptor layer side. Next, a patch (application area of the formulation: 5 cm²) was attached to the stratum corneum side of the skin. Normal saline was supplied to the receptor layer, and sampling of the receptor solution was carried out at a speed of 5mL/hour every 2 hours up to 24 hours. For receptor solutions obtained at the respective times, the flow volume was measured, and drug concentration was measured using high-performance liquid chromatography. From the obtained measurement values, the permeation rate for 1 hour was calculated to obtain a drug permeation rate per unit area of the skin at a steady state. The respective maximum values of the drug permeation rate (maximum skin permeation rate) obtained in the period from the start of the test to 24 hours are listed in Tables 1-3.

### (Patch property test)

For the respective patches obtained in Examples 1-6 and Comparative Examples 1-10, adhesion properties, cohesion properties and stability of the adhesive layer were evaluated. The evaluation test for the adhesion property was conducted using a probe tack tester and a peel testing machine. The evaluation test for the cohesion property was conducted using a creep measuring machine. The stability was evaluated by conducting the aforementioned adhesion property test for a patch that had been stored at 40 °C for 6 months. Evaluation of these patch properties was conducted on the basis of the following standard:
A: very good
B: good
C: bad.
The obtained results are listed in Tables 1-3.

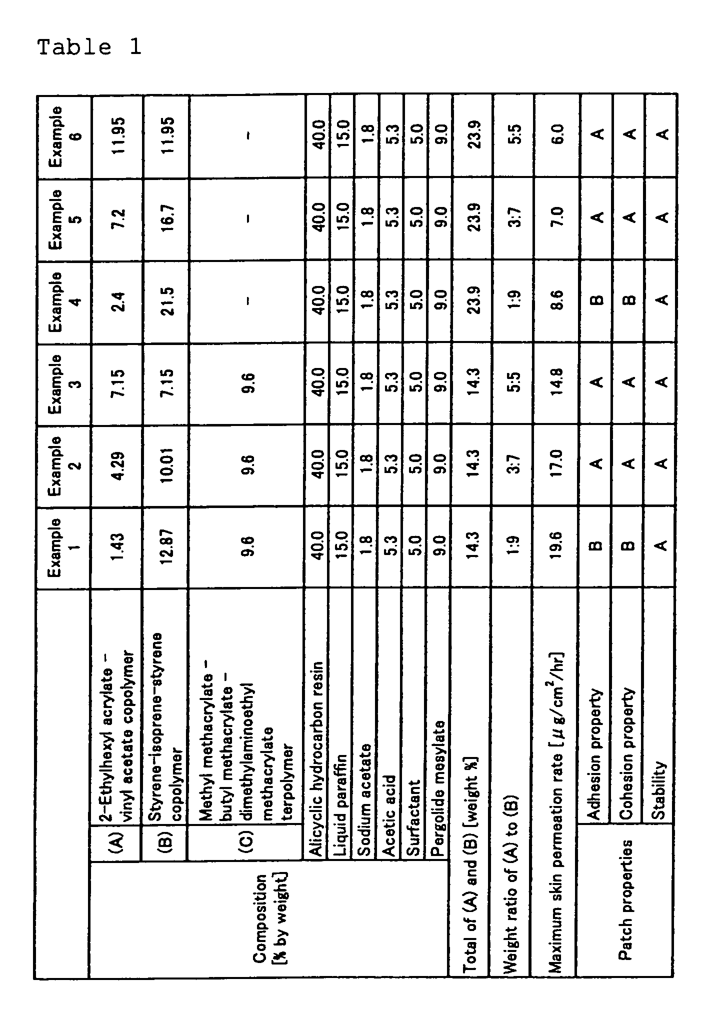

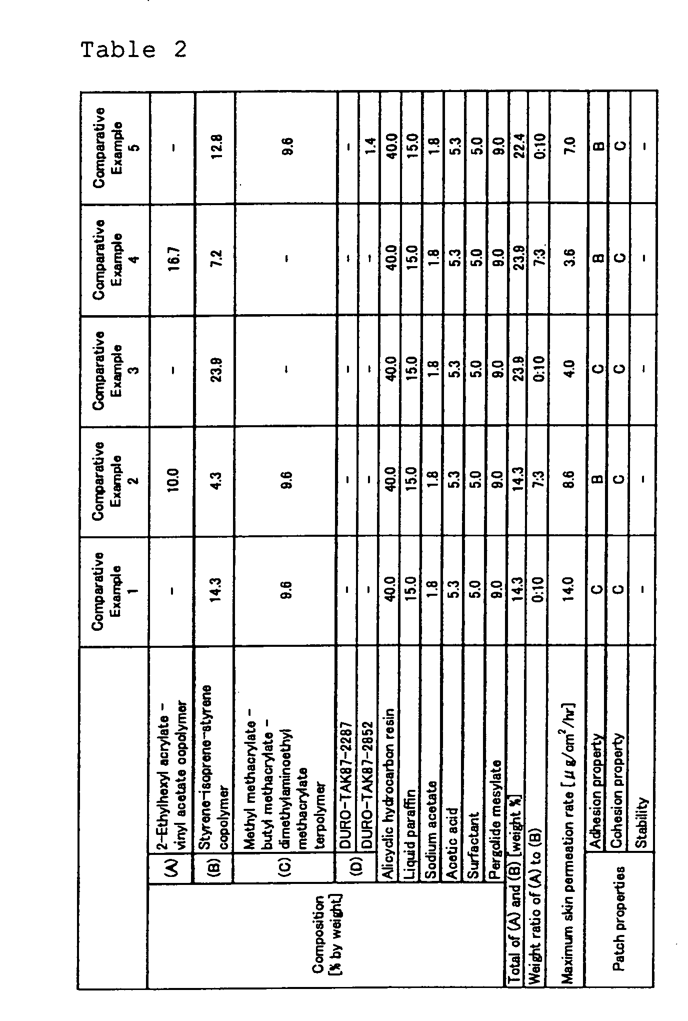

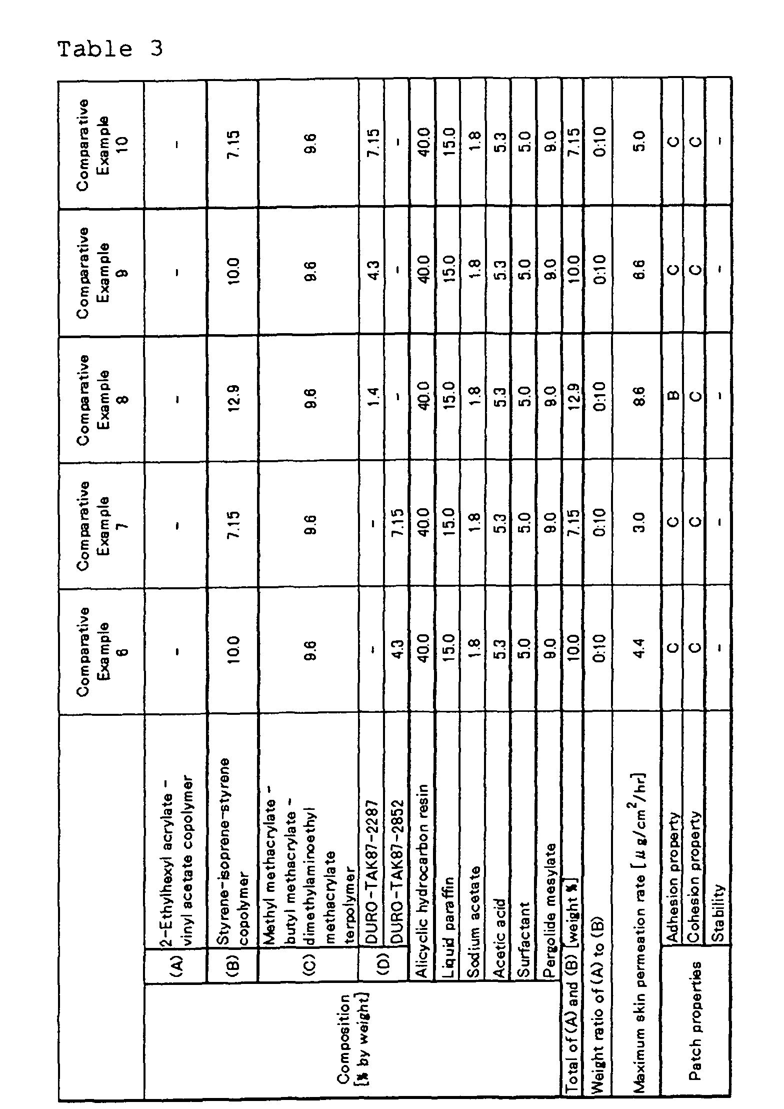

### Industrial Applicability

As described above, according to the present invention, for a patch containing pergolide and/or a pharmaceutically acceptable salt thereof as a drug, a high level can be attained for both the skin absorption properties of the drug and the patch properties.

## Claims

1. A patch comprising a backing layer and an adhesive layer disposed on the backing layer and compounded with an adhesive base agent and pergolide and/or a pharmaceutically acceptable salt thereof, wherein the adhesive base agent comprises an acrylic polymer including no substantial carboxyl group and hydroxyl group in the molecule and having self-adhesion properties and a rubber polymer, and a weight ratio of the content of the acrylic polymer to the content of the rubber polymer is from 1:1 to 1:9.

2. The patch according to claim 1, wherein the adhesive base agent further comprises a basic nitrogen-including polymer including a basic nitrogen and having no self-adhesion property, and a weight ratio of the total content of the acrylic polymer and the rubber polymer to the content of the basic nitrogen-including polymer is from 9:1 to 1:1.

3. The patch according to claim 2, wherein the basic nitrogen-including polymer is at least one kind selected from methyl methacrylate - butyl methacrylate - dimethylaminoethyl methacrylate terpolymer and polyvinyl acetal diethylamino acetate.

4. The patch according to any of claims 1-3, wherein the adhesive layer further comprises an alicyclic saturated hydrocarbon resin-based tackifier, and a weight ratio of the total content of acrylic polymer and the rubber polymer to the content of the tackifier is from 1:1 to 1:9.

5. The patch according to any of claims 1-4, wherein the acrylic polymer is at least one kind selected from:
copolymer of polyacrylate including at least one kind selected from 2-ethylhexyl acrylate, butyl acrylate, diacetone acrylamide and tetraethylene glycol dimethacrylate and polymethyl methacrylate;
2-ethylhexyl acrylate - N-vinyl-2-pyrrolidone - 1,6-hexane glycol dimethacrylate terpolymer; and
2-ethylhexyl acrylate - vinyl acetate copolymer.

6. The patch according to any of claims 1-5, wherein the rubber polymer is at least one kind selected from styrene-isoprene-styrene block copolymer, styrene-butadiene-styrene block copolymer, styrene-butadiene rubber, polyisobutylene, isoprene rubber and silicone rubber.

7. The patch according to any of claims 1-6, wherein the rubber polymer is styrene-isoprene-styrene block copolymer.

8. The patch according to any of claims 1-7, wherein the acrylic polymer is at least one kind selected from 2-ethylhexyl acrylate - N-vinyl-2-pyrrolidone - 1,6-hexane glycol dimethacrylate terpolymer and 2-ethylhexyl acrylate - vinyl acetate copolymer, and the rubber polymer is styrene-isoprene-styrene block copolymer.

9. The patch according to any of claims 1-8, wherein the adhesive layer is compounded with mesylate salt of pergolide.

10. The patch according to any of claims 1-9, wherein the adhesive layer further comprises an organic acid.

11. The patch according to claim 10, wherein the organic acid is acetic acid and/or a pharmaceutically acceptable salt thereof.
